# EUROPÄISCHE PATENTSCHRIFT

(11) **EP 2 115 014 B1**
(45) Veröffentlichungstag und Bekanntmachung des Hinweises auf die Patenterteilung: **16.01.2019**
(21) Anmeldenummer: 08708656.7
(22) Anmeldetag: 04.02.2008
(51) Int. Cl.: C08F 2/00, C08F 2/10, C08F 2/16, C08F 2/18, A61L 15/60, C08F 6/00

(54) **VERFAHREN ZUR HERSTELLUNG WASSERABSORBIERENDER POLYMERPARTIKEL DURCH POLYMERISATION VON TROPFEN EINER MONOMERLÖSUNG**
METHOD FOR PRODUCING WATER-ABSORBENT POLYMER PARTICLES BY THE POLYMERISATION OF DROPLETS OF A MONOMER SOLUTION
PROCÉDÉ DE PRODUCTION DE PARTICULES POLYMÈRES HYDROABSORBANTES PAR POLYMÉRISATION DE GOUTTES D'UNE SOLUTION MONOMÈRE

(30) Priorität: 06.02.2007 EP 07101834
(43) Veröffentlichungstag der Anmeldung: 11.11.2009
(73) Patentinhaber: BASF SE, 67056 Ludwigshafen am Rhein (DE)
(72) Erfinder: LÖSCH, Dennis, 67122 Altrip (DE); KRÜGER, Marco, 68161 Mannheim (DE); BLEI, Stefan, 68163 Mannheim (DE); WEISMANTEL, Matthias, 63637 Jossgrund (DE); HEIDE, Wilfried, 67251 Freinsheim (DE)
(74) Vertreter: BASF IP Association
(86) Internationale Anmeldenummer: PCT/EP2008/051353
(87) Internationale Veröffentlichungsnummer: WO 2008/095901

(56) Entgegenhaltungen:
- EP-A- 1 690 887
- EP-A2- 0 349 240
- WO-A-2006/079631
- WO-A1-2008/068208
- US-A- 5 866 678

## Beschreibung

Die vorliegende Erfindung betrifft ein Verfahren zur Herstellung wasserabsorbierender Polymerpartikel durch Polymerisation von Tropfen einer Monomerlösung in einer umgebenden Gasphase, wobei die erhaltenen Polymerpartikel einen Wassergehalt von mindestens 5 Gew.-% aufweisen und in fluidisiertem Zustand in Gegenwart von Wasserdampf bei einer Temperatur von mindestens 60°C thermisch nachbehandelt werden.

Die Herstellung wasserabsorbierender Polymerpartikel wird in der Monographie "Modern Superabsorbent Polymer Technology", F.L. Buchholz und A.T. Graham, Wiley-VCH, 1998, Seiten 71 bis 103, beschrieben.

Wasserabsorbierende Polymere werden als wässrige Lösungen absorbierende Produkte zur Herstellung von Windeln, Tampons, Damenbinden und anderen Hygieneartikeln, aber auch als wasserzurückhaltende Mittel im landwirtschaftlichen Gartenbau verwendet.

Durch Sprühpolymerisation konnten die Verfahrensschritte Polymerisation und Trocknung zusammengefasst werden. Zusätzlich konnte die Partikelgröße durch geeignete Verfahrensführung in gewissen Grenzen eingestellt werden.

Die Herstellung wasserabsorbierender Polymerpartikel durch Polymerisation von Tropfen einer Monomerlösung wird beispielsweise in EP 348 180 A1, WO 96/40427 A1, US 5,269,980, DE 103 14 466 A1, DE 103 40 253 A1 und DE 10 2004 024 437 A1, WO 2006/077054 A1 sowie der älteren deutschen Anmeldung mit dem Aktenzeichen 102006001596.7 und der älteren PCT-Anmeldung mit dem Aktenzeichen PCT/EP2006/062252 beschrieben.

Aufgabe der vorliegenden Erfindung war die Bereitstellung eines verbesserten Verfahrens zur Herstellung wasserabsorbierender Polymerpartikel durch Polymerisation von Tropfen einer Monomerlösung in einer die Tropfen umgebenden Gasphase.

Insbesondere war es eine Aufgabe der vorliegenden Erfindung ein Verfahren bereitzustellen, das wasserabsorbierende Polymerpartikel mit wenig Restmonomeren erzeugt.

Gelöst wurde die Aufgabe durch ein Verfahren zur Herstellung wasserabsorbierender Polymerpartikel durch Polymerisation von Tropfen einer Monomerlösung, enthaltend
a) mindestens ein ethylenisch ungesättigtes Monomer,
b) wahlweise einen Vernetzer,
c) mindestens einen Initiator und
d) Wasser,
in einer umgebenden Gasphase, dadurch gekennzeichnet, dass die erhaltenen Polymerpartikel einen Wassergehalt von mindestens 5 Gew.-% aufweisen, in fluidisiertem Zustand in Gegenwart eines Gasstromes bei einer Temperatur von mindestens 60°C thermisch nachbehandelt werden und die Nachbehandlung mindestens 5 Minuten durchgeführt wird, wobei der Gasstrom bei einer Temperatur von weniger als 100°C eine relative Feuchte von mindestens 20% aufweist oder bei einer Temperatur von 100°C oder mehr mindesten 0,25 kg Wasserdampf pro kg trockenes Gas enthält, wobei die relative Feuchte der Quotient aus Wasserdampfpartialdruck und Wasserdampfdruck bei Sättigung bei einer gegebenen Temperatur multipliziert mit 100% ist, und die Nachbehandlungstemperatur und die relative Feuchte des Gasstromes so gewählt werden, dass sich der Wassergehalt der Polymerpartikel während der Nachbehandlung um weniger als 40% ändert, wobei die Änderung die relative Änderung bedeutet.

Der Wassergehalt der erhaltenen Polymerpartikel beträgt vorzugsweise von 8 bis 40 Gew.-%, besonders bevorzugt von 10 bis 30 Gew.-%, ganz besonders bevorzugt von 12 bis 20 Gew.-%.
Die Temperatur bei der thermischen Nachbehandlung beträgt vorzugsweise von 70 bis 150°C, besonders bevorzugt von 80 bis 140°C, ganz besonders von 90 bis 130°C. Der Wassergehalt der Polymerpartikel ändert sich während der thermischen Nachbehandlung vorzugsweise um weniger als 20 %, ganz besonders bevorzugt um weniger als 10 %, wobei die Änderung die relative Änderung bedeutet. Beispielsweise kann bei einer festgelegten Nachbehandlungstemperatur die Änderung des Wassergehalts der Polymerpartikel vor und nach der Nachbehandlung gemessen und die relative Feuchte des Gasstromes entsprechend angepasst werden.
Die thermische Nachbehandlung wird vorzugsweise von 5 bis 120 Minuten, besonders bevorzugt von 8 bis 60 Minuten, ganz besonders bevorzugt von 10 bis 30 Minuten, durchgeführt.
Bei Temperaturen von weniger als 100°C beträgt die relative Feuchte des Gasstromes vorzugsweise von 50 bis 98%, besonders bevorzugt von 70 bis 96%, ganz besonders bevorzugt von 85 bis 95%. Die relative Feuchte ist der Quotient aus Wasserdampfpartialdruck und Wasserdampfdruck (Sättigung) bei einer gegebenen Temperatur multipliziert mit 100%.
Bei Temperaturen von 100°C oder mehr beträgt der Wasserdampfgehalt des Gases vorzugsweise von 1 bis 10 kg pro kg trockenes Gas, besonders bevorzugt von 2 bis 7,5 kg pro kg trockenes Gas, ganz besonders bevorzugt von 3 bis 5 kg pro kg trockenes Gas.

Die übrigen Bestandteile des Gases sind vorzugsweise Stickstoff, Luft oder Luft/Stickstoff-Gemische, besonders bevorzugt Stickstoff oder Luft/Stickstoff-Gemische mit weniger als 10 Vol.-% Sauerstoff.

Im fluidisierten Zustand ist die kinetische Energie der Polymerpartikel größer als das Kohäsions- bzw. Adhäsionspotential zwischen den Polymerpartikeln.

Der fluidisierte Zustand kann durch ein Wirbelbett erreicht werden. Dabei werden die wasserabsorbierenden Polymerpartikel von unten angeströmt, so dass die Partikel eine Wirbelschicht ausbilden. Die Höhe der Wirbelschicht wird durch Gasmenge und Gasgeschwindigkeit, d.h. über den Druckverlust der Wirbelschicht (kinetische Energie des Gases), eingestellt.

Die Geschwindigkeit des Gasstromes beträgt vorzugsweise von 0,5 bis 2,5 m/s, besonders bevorzugt von 0,8 bis 1,5 m/s, ganz besonders bevorzugt von 0,9 bis 1,2 m/s.

Die vorliegenden Erfindung liegt die Erkenntnis zugrunde, dass sich Restmonomere in den durch Polymerisation von Tropfen einer Monomerlösung erhaltenen Polymerpartikeln durch Kontakt mit einem strömenden Gas entfernen lassen. Die Restmonomeren lassen sich bei höheren Temperaturen und längeren Verweilzeiten besser entfernen. Wichtig ist hierbei, dass die Polymerpartikel nicht zu trocken sind. Bei zu trockenen Partikeln nehmen die Restmonomeren nur unwesentlich ab. Ein zu hoher Wassergehalt erhöht die Verbackungsneigung der Polymerpartikel. Damit die wasserabsorbierenden Polymerpartikel während der thermischen Nachbehandlung nicht zu schnell trocknen muss das anströmende Gas bereits Wasserdampf enthalten.

Während der thermischen Nachbehandlung sollte daher der Wassergehalt der Polymerpartikel im Bereich von vorzugsweise 5 bis 40 Gew.-%, besonders bevorzugt von 8 bis 30 Gew.-%, ganz besonders von 10 bis 20 Gew.-%, liegen.

Die Monomeren a) sind vorzugsweise wasserlöslich, d.h. die Löslichkeit in Wasser bei 23°C beträgt typischerweise mindestens 1 g/100 g Wasser, vorzugsweise mindestens 5 g/100 g Wasser, besonders bevorzugt mindestens 25 g/100 g Wasser, ganz besonders bevorzugt mindestens 50 g/100 g Wasser, und haben vorzugsweise mindestens je eine Säuregruppe.

Geeignete Monomere a) sind beispielsweise ethylenisch ungesättigte Carbonsäuren, wie Acrylsäure, Methacrylsäure, Maleinsäure, Fumarsäure und Itaconsäure. Besonders bevorzugte Monomere sind Acrylsäure und Methacrylsäure. Ganz besonders bevorzugt ist Acrylsäure.

Die bevorzugten Monomere a) haben mindestens eine Säuregruppe, wobei die Säuregruppen vorzugsweise zumindest teilweise neutralisiert sind.

Der Anteil an Acrylsäure und/oder deren Salzen an der Gesamtmenge der Monomeren a) beträgt vorzugsweise mindestens 50 mol-%, besonders bevorzugt mindestens 90 mol-%, ganz besonders bevorzugt mindestens 95 mol-%.

Die Säuregruppen der Monomere a) sind üblicherweise teilweise neutralisiert, vorzugsweise zu 25 bis 85 mol-%, bevorzugt zu 50 bis 80 mol-%, besonders bevorzugt 60 bis 75 mol-%, wobei die üblichen Neutralisationsmittel verwendet werden können, vorzugsweise Alkalimetallhydroxide, Alkalimetalloxide, Alkalimetallcarbonate oder Alkalimetallhydrogencarbonate sowie deren Mischungen. Statt Alkalimetallsalzen können auch Ammoniumsalze verwendet werden. Natrium und Kalium sind als Alkalimetalle besonders bevorzugt, ganz besonders bevorzugt sind jedoch Natriumhydroxid, Natriumcarbonat oder Natriumhydrogencarbonat sowie deren Mischungen. Üblicherweise wird die Neutralisation durch Einmischung des Neutralisationsmittels als wässrige Lösung, als Schmelze, oder bevorzugt auch als Feststoff erreicht. Beispielsweise kann Natriumhydroxid mit einem Wasseranteil deutlich unter 50 Gew.-% als wachsartige Masse mit einem Schmelzpunkt oberhalb 23°C vorliegen. In diesem Fall ist eine Dosierung als Stückgut oder Schmelze bei erhöhter Temperatur möglich.

Die Monomere a), insbesondere Acrylsäure, enthalten vorzugsweise bis zu 0,025 Gew.-% eines Hydrochinonhalbethers. Bevorzugte Hydrochinonhalbether sind Hydrochinonmonomethylether (MEHQ).

Die Monomerlösung enthält vorzugsweise höchstens 160 Gew.-ppm, bevorzugt höchstens 130 Gew.-ppm, besonders bevorzugt höchstens 70 Gew.-ppm, bevorzugt mindesten 10 Gew.-ppm, besonders bevorzugt mindesten 30 Gew.-ppm, insbesondere um 50 Gew.-ppm, Hydrochinonhalbether, jeweils bezogen auf Acrylsäure, wobei Acrylsäuresalze als Acrylsäure mit berücksichtigt werden. Beispielsweise kann zur Herstellung der Monomerlösung eine Acrylsäure mit einem entsprechenden Gehalt an Hydrochinonhalbether verwendet werden.

Die Polymerisationsinhibitoren können aber auch durch Absorption, beispielsweise an Aktivkohle, aus der Monomerlösung entfernt werden.

Vernetzer b) sind Verbindungen mit mindestens zwei polymerisierbaren Gruppen, die in das Polymernetzwerk radikalisch einpolymerisiert werden können. Geeignete Vernetzer b) sind beispielsweise Ethylenglykoldimethacrylat, Diethylenglykoldiacrylat, Allylmethacrylat, Trimethylolpropantriacrylat, Triallylamin, Tetraallyloxyethan, wie in EP 530 438 A1 beschrieben, Di- und Triacrylate, wie in EP 547 847 A1, EP 559 476 A1, EP 632 068 A1, WO 93/21237 A1, WO 2003/104299 A1, WO 2003/104300 A1, WO 2003/104301 A1 und DE 103 31 450 A1 beschrieben, gemischte Acrylate, die neben Acrylatgruppen weitere ethylenisch ungesättigte Gruppen enthalten, wie in DE 103 31 456 A1 und DE 103 55 401 A1 beschrieben, oder Vernetzermischungen, wie beispielsweise in DE 195 43 368 A1, DE 196 46 484 A1, WO 90/15830 A1 und WO 2002/32962 A2 beschrieben.

Geeignete Vernetzer b) sind insbesondere N,N'-Methylenbisacrylamid und N,N'-Methylenbismethacrylamid, Ester ungesättigter Mono- oder Polycarbonsäuren von Polyolen, wie Diacrylat oder Triacrylat, beispielsweise Butandiol- oder Ethylenglykoldiacrylat bzw. -methacrylat sowie Trimethylolpropantriacrylat und Allylverbindungen, wie Allyl(meth)acrylat, Triallylcyanurat, Maleinsäurediallylester, Polyallylester, Tetraallyloxyethan, Triallylamin, Tetraallylethylendiamin, Allylester der Phosphorsäure sowie Vinylphosphonsäurederivate, wie sie beispielsweise in EP 343 427 A2 beschrieben sind. Weiterhin geeignete Vernetzer b) sind Pentaerythritoldi-, Pentaerythritoltri- und Pentaerythritoltetraallylether, Polyethylenglykoldiallylether, Ethylenglykoldiallylether, Glyzerindi- und Glyzerintriallylether, Polyallylether auf Basis Sorbitol, sowie ethoxylierte Varianten davon. Im erfindungsgemäßen Verfahren einsetzbar sind Di(meth)acrylate von Polyethylenglykolen, wobei das eingesetzte Polyethylenglykol ein Molekulargewicht zwischen 300 und 1000 aufweist.

Besonders vorteilhafte Vernetzer b) sind jedoch Di- und Triacrylate des 3- bis 20-fach ethoxylierten Glyzerins, des 3- bis 20-fach ethoxylierten Trimethylolpropans, des 3- bis 20-fach ethoxylierten Trimethylolethans, insbesondere Di- und Triacrylate des 2- bis 6-fach ethoxylierten Glyzerins oder Trimethylolpropans, des 3-fach propoxylierten Glyzerins oder Trimethylolpropans, sowie des 3-fach gemischt ethoxylierten oder propoxylierten Glyzerins oder Trimethylolpropans, des 15-fach ethoxylierten Glyzerins oder Trimethylolpropans, sowie des mindestens 40-fach ethoxylierten Glyzerins, Trimethylolethans oder Trimethylolpropans.

Ganz besonders bevorzugte Vernetzer b) sind die mit Acrylsäure oder Methacrylsäure zu Di- oder Triacrylaten veresterten mehrfach ethoxylierten und/oder propoxylierten Glyzerine, wie sie beispielsweise in WO 2003/104301 A1 beschrieben sind. Besonders vorteilhaft sind Di- und/oder Triacrylate des 3- bis 10-fach ethoxylierten Glyzerins. Ganz besonders bevorzugt sind Di- oder Triacrylate des 1- bis 5-fach ethoxylierten und/oder propoxylierten Glyzerins. Am meisten bevorzugt sind die Triacrylate des 3-bis 5-fach ethoxylierten und/oder propoxylierten Glyzerins.

Die Monomerlösung enthält vorzugsweise mindestens 0,1 Gew.-%, bevorzugt mindestens 0,2 Gew.-%, besonders bevorzugt mindestens 0,3 Gew.-%, ganz besonders bevorzugt mindestens 0,4 Gew.-%, Vernetzer b), jeweils bezogen auf Monomer a).

Als Initiatoren c) können sämtliche unter den Polymerisationsbedingungen in Radikale zerfallende Verbindungen eingesetzt werden, beispielsweise Peroxide, Hydroperoxide, Wasserstoffperoxid, Persulfate, Azoverbindungen und die sogenannten Redoxinitiatoren. Bevorzugt ist der Einsatz von wasserlöslichen Initiatoren. In manchen Fällen ist es vorteilhaft, Mischungen verschiedener Initiatoren zu verwenden, beispielsweise Mischungen aus Wasserstoffperoxid und Natrium- oder Kaliumperoxodisulfat. Mischungen aus Wasserstoffperoxid und Natriumperoxodisulfat können in jedem beliebigen Verhältnis verwendet werden.

Besonders bevorzugte Initiatoren c) sind Azoinitiatoren, wie 2,2'-Azobis[2-(2-imidazolin-2-yl)propan]dihydrochlorid und 2,2'-Azobis[2-(5-methyl-2-imidazolin-2-yl)propan]dihydrochlorid, und Photoinitiatoren, wie 2-Hydroxy-2-methylpropiophenon und 1-[4-(2-Hydroxyethoxy)-phenyl]-2-hydroxy-2-methyl-1-propan-1-on, Redoxinitiatoren, wie Natriumpersulfat/ Hydroxymethylsulfinsäure, Ammoniumperoxodisulfat/Hydroxymethylsulfinsäure, Wasserstoffperoxid/Hydroxymethylsulfinsäure, Natriumpersulfat/Ascorbinsäure, Ammoniumperoxodisulfat/Ascorbinsäure und Wasserstoffperoxid/Ascorbinsäure, Photoinitiatoren, wie 1-[4-(2-Hydroxyethoxy)-phenyl]-2-hydroxy-2-methyl-1-propan-1-on, sowie deren Mischungen.

Die Initiatoren werden in üblichen Mengen eingesetzt, beispielsweise in Mengen von 0,001 bis 5 Gew.-%, vorzugsweise 0,01 bis 2 Gew.-%, bezogen auf die Monomeren a).

In einer bevorzugten Ausführungsform der vorliegenden Erfindung werden mindestens eine Azoverbindung und mindestens ein Persulfat als Initiatoren c) eingesetzt.

Die Menge an Azoverbindung beträgt vorzugsweise mindestens 0,1 Gew.-%, besonders bevorzugt mindestens 0,25 Gew.-%, ganz besonders bevorzugt mindestens 0,5 Gew.-%, bezogen auf die Monomeren a).

Die Menge an Persulfat beträgt vorzugsweise mindestens 0,25 Gew.-%, besonders bevorzugt mindestens 0,5 Gew.-%, ganz besonders bevorzugt mindestens 0,75 Gew.-%, bezogen auf die Monomeren a).

Die Azoverbindungen zerfallen üblicherweise schnell in Radikale. Im Gegensatz dazu sind Persulfate relativ langsame Polymerisationsinitiatoren. Dies bedeutet, dass die durch Polymerisation von Tropfen einer Monomerlösung erhaltenen wasserabsorbierenden Polymerpartikel noch signifikante Mengen an Persulfat enthalten. Diese Persulfate zerfallen während der thermischen Nachbehandlung und vermindern damit zusätzlich die Restmonomeren.

Gleichzeitig bewirkten die während der thermischen Nachbehandlung zerfallenden Persulfate eine mehr oder weniger starke Abnahme der Vernetzungsdichte, wodurch die Zentrifugenretentionskapazität (CRC) steigt und die Absorption unter Druck (AUL0.7psi) fällt. Dieser Effekt kann beispielsweise durch eine höhere Einsatzmenge an Vernetzer b) kompensiert werden.

Der Feststoffgehalt der Monomerlösung beträgt vorzugsweise mindestens 35 Gew.-%, bevorzugt mindestens 38 Gew.-%, besonders bevorzugt mindestens 40 Gew.-%, ganz besonders bevorzugt mindestens 42 Gew.-%. Dabei ist der Feststoffgehalt die Summe aller nach der Polymerisation nichtflüchtigen Bestandteile. Dies sind Monomer a), Vernetzer b) und Initiator c).

Der Sauerstoffgehalt der Monomerlösung beträgt vorzugsweise mindestens 1 Gew.-ppm, besonders bevorzugt mindestens 2 Gew.-ppm, ganz besonders bevorzugt mindestens 5 Gew.-ppm. Auf die übliche Inertisierung der Monomerlösung kann daher weitgehend verzichtet werden.

Der erhöhte Sauerstoffgehalt stabilisiert die Monomerlösung und ermöglicht die Verwendung geringerer Mengen an Polymerisationsinhibitor und vermindert damit die durch den Polymerisationsinhibitor verursachten Produktverfärbungen.

Die Monomerlösung wird zur Polymerisation in die Gasphase dosiert. Der Sauerstoffgehalt der Gasphase beträgt vorzugsweise 0,001 bis 0,15 Vol.-%, besonders bevorzugt 0,002 bis 0,1 Vol.-%, ganz besonders bevorzugt 0,005 bis 0,05 Vol.-%.

Die Gasphase enthält neben Sauerstoff vorzugsweise nur inerte Gase, d.h. Gase, die unter Reaktionsbedingungen nicht in die Polymerisation eingreifen, beispielsweise Stickstoff und/oder Wasserdampf.

Die Monomerlösung wird unter Ausbildung von Tropfen in die Gasphase dosiert. Die Tropfen können beispielsweise mittels einer Vertropferplatte erzeugt werden.

Eine Vertropferplatte ist eine Platte mit mindestens einer Bohrung, wobei die Flüssigkeit von oben durch die Bohrung tritt. Die Vertropferplatte bzw. die Flüssigkeit kann in Schwingungen versetzt werden, wodurch an der Unterseite der Vertropferplatte je Bohrung eine idealerweise monodisperse Tropfenkette erzeugt wird. In einer bevorzugten Ausführungsform wird die Vertropferplatte nicht angeregt.

Die Anzahl und die Größe der Bohrungen werden gemäß der gewünschten Kapazität und Tropfengröße ausgewählt. Der Tropfendurchmesser beträgt dabei üblicherweise das 1,9fache des Durchmessers der Bohrung. Wichtig ist hierbei, dass die zu vertropfende Flüssigkeit nicht zu schnell durch die Bohrung tritt bzw. der Druckverlust über die Bohrung nicht zu groß ist. Ansonsten wird die Flüssigkeit nicht vertropft, sondern der Flüssigkeitsstrahl wird infolge der hohen kinetischen Energie zerrissen (versprüht). Der Vertropfer wird im Strömungsbereich des laminaren Strahlzerfalls betrieben, d.h. die Reynoldszahl bezogen auf den Durchsatz pro Bohrung und den Bohrungsdurchmesser ist vorzugsweise kleiner als 2.000, bevorzugt kleiner 1.000, besonders bevorzugt kleiner 500, ganz besonders bevorzugt kleiner 100. Der Druckverlust über die Bohrung beträgt vorzugsweise weniger als 2,5 bar, besonders bevorzugt weniger als 1,5 bar, ganz besonders bevorzugt weniger als 1 bar.
Die Vertropferplatte weist üblicherweise mindestens eine, vorzugsweise mindestens 10, besonders bevorzugt mindestens 50, und üblicherweise bis zu 10.000, vorzugsweise bis zu 5.000, besonders bevorzugt bis zu 1.000, Bohrungen auf, wobei die Bohrungen üblicherweise gleichmäßig über die Vertropferplatte verteilt sind, vorzugsweise in der sogenannten Dreiecksteilung, d.h. jeweils drei Bohrungen bilden die Ecken eines gleichseitigen Dreiecks. Der Durchmesser der Bohrungen wird an die gewünschte Tropfengröße angepasst.

Die Tropfen können aber auch mittels pneumatischer Ziehdüsen, Rotation, Zerschneiden eines Strahls oder schnell ansteuerbarer Mikroventildüsen erzeugt werden.

In einer pneumatischen Ziehdüse wird ein Flüssigkeitsstrahl zusammen mit einem Gasstrom durch eine Blende beschleunigt. Über die Gasmenge kann der Durchmesser des Flüssigkeitsstrahls und damit der Tropfendurchmesser beeinflusst werden.

Bei der Tropfenerzeugung durch Rotation tritt die Flüssigkeit durch die Öffnungen einer rotierenden Scheibe. Durch die auf die Flüssigkeit wirkende Fliehkraft werden Tropfen definierter Größe abgerissen. Bevorzugte Vorrichtungen zur Rotationsvertropfung werden beispielsweise in DE 43 08 842 A1 beschrieben

Der austretende Flüssigkeitsstrahl kann aber auch mittels eines rotierenden Messers in definierte Segmente zerschnitten werden. Jedes Segment bildet anschließend einen Tropfen.

Bei Verwendung von Mikroventildüsen werden direkt Tropfen mit definiertem Flüssigkeitsvolumen erzeugt.

Die erzeugten Tropfen weisen einen mittleren Durchmesser von vorzugsweise mindestens 200 µm, besonders bevorzugt von mindestens 250 µm, ganz besonders bevorzugt von mindestens 300 µm, auf, wobei der Tropfendurchmesser durch Lichtstreuung bestimmt werden kann und den volumengemittelten mittleren Durchmesser bedeutet.

Der Polymerisationsreaktor wird von einem Gas durchströmt. Dabei kann das Trägergas im Gleichstrom oder im Gegenstrom zu den frei fallenden Tropfen der Monomerlösung durch den Reaktionsraum geführt werden, bevorzugt im Gleichstrom, d.h. von unten nach oben. Vorzugsweise wird das Gas nach einem Durchgang zumindest teilweise, bevorzugt zu mindestens 50%, besonders bevorzugt zu mindestens 75%, als Kreisgas in den Reaktionsraum zurückgeführt. Üblicherweise wird eine Teilmenge des Trägergases nach jedem Durchgang ausgeschleust, vorzugsweise bis zu 10%, besonders bevorzugt bis zu 3%, ganz besonders bevorzugt bis zu 1%.

Die Gasgeschwindigkeit wird vorzugsweise so eingestellt, dass die Strömung im Polymerisationsreaktor gerichtet ist, beispielsweise liegen keine der allgemeinen Strömungsrichtung entgegengesetzte Konvektionswirbel vor, und beträgt beispielsweise 0,01 bis 5 m/s, vorzugsweise 0,02 bis 4 m/s, besonders bevorzugt 0,05 bis 3 m/s, ganz besonders bevorzugt 0,1 bis 2 m/s.

Das den Reaktor durchströmende Gas wird zweckmäßigerweise vor dem Reaktor auf die Reaktionstemperatur vorgewärmt.

Die Reaktionstemperatur beträgt bei der thermisch induzierten Polymerisation vorzugsweise 100 bis 250°C, besonders bevorzugt 120 bis 200°C, ganz besonders bevorzugt 150 bis 180°C.

Die Reaktion kann im Überdruck oder im Unterdruck durchgeführt werden, ein Unterdruck von bis zu 100 mbar gegenüber dem Umgebungsdruck ist bevorzugt.

Das Reaktionsabgas, d.h. das der Reaktionsraum verlassende Gas, kann beispielsweise in einem Wärmeaustauscher abgekühlt werden. Dabei kondensieren Wasser und nicht umgesetztes Monomer a). Danach kann das Reaktionsabgas zumindest teilweise wieder aufgewärmt und als Kreisgas in den Reaktor zurückgeführt werden. Ein Teil des Reaktionsabgases kann ausgeschleust und durch frisches Gas ersetzt werden, wobei im Reaktionsabgas enthaltenes Wasser und nicht umgesetzte Monomere a) abgetrennt und rückgeführt werden können.

Besonders bevorzugt ist ein Wärmeverbund, dass heißt, ein Teil der Abwärme beim Abkühlen des Abgases wird zum Aufwärmen des Kreisgases verwendet.

Die Reaktoren können begleitbeheizt werden. Die Begleitheizung wird dabei so eingestellt, dass die Wandtemperatur mindestens 5°C oberhalb der Reaktorinnentemperatur liegt und die Kondensation an den Reaktorwänden zuverlässig vermieden wird.

Das Reaktionsprodukt wird anschließend thermisch nachbehandelt und wahlweise getrocknet, vorzugsweise in mindestens einer Wirbelschicht.

Die Polymerpartikel können zur weiteren Verbesserung der Eigenschaften nachvernetzt werden.

Nachvernetzer sind Verbindungen, die mindestens zwei Gruppen enthalten, die mit den Carboxylatgruppen des Hydrogels kovalente Bindungen bilden können. Geeignete Verbindungen sind beispielsweise Alkoxysiliylverbindungen, Polyaziridine, Polyamine, Polyamidoamine, Di- oder Polyepoxide, wie in EP 83 022 A2, EP 543 303 A1 und EP 937 736 A2 beschrieben, di- oder polyfunktionelle Alkohole, wie in DE 33 14 019 A1, DE 35 23 617 A1 und EP 450 922 A2 beschrieben, oder ß-Hydroxyalkylamide, wie in DE 102 04 938 A1 und US 6,239,230 beschrieben.

Des weiteren sind in DE 40 20 780 C1 zyklische Karbonate, in DE 198 07 502 A1 2-Oxazolidon und dessen Derivate, wie 2-Hydroxyethyl-2-oxazolidon, in DE 198 07 992 C1 Bis- und Poly-2-oxazolidinone, in DE 198 54 573 A1 2-Oxotetrahydro-1,3-oxazin und dessen Derivate, in DE 198 54 574 A1 N-Acyl-2-Oxazolidone, in DE 102 04 937 A1 zyklische Harnstoffe, in DE 103 34 584 A1 bizyklische Amidacetale, in EP 1 199 327 A2 Oxetane und zyklische Harnstoffe und in WO 2003/31482 A1 Morpholin-2,3-dion und dessen Derivate als geeignete Nachvernetzer beschrieben.

Die Menge an Nachvernetzer beträgt vorzugsweise 0,01 bis 1 Gew.-%, besonders bevorzugt 0,05 bis 0,5 Gew.-%, ganz besonders bevorzugt 0,1 bis 0,2 Gew.-%, jeweils bezogen auf das Polymer.

Die Nachvernetzung wird üblicherweise so durchgeführt, dass eine Lösung des Nachvernetzers auf das Hydrogel oder die trockenen Polymerpartikel aufgesprüht wird. Im Anschluss an das Aufsprühen wird thermisch getrocknet, wobei die Nachvernetzungsreaktion sowohl vor als auch während der Trocknung stattfinden kann.

Das Aufsprühen einer Lösung des Vernetzers wird vorzugsweise in Mischern mit bewegten Mischwerkzeugen, wie Schneckenmischer, Paddelmischer, Scheibenmischer, Pflugscharmischer und Schaufelmischer, durchgeführt werden. Besonders bevorzugt sind Vertikalmischer, ganz besonders bevorzugt sind Pflugscharmischer und Schaufelmischer. Geeignete Mischer sind beispielsweise Lödige-Mischer, Bepex-Mischer, Nauta-Mischer, Processall-Mischer und Schugi-Mischer.

Die thermische Trocknung wird vorzugsweise in Kontakttrocknern, besonders bevorzugt Schaufeltrocknern, ganz besonders bevorzugt Scheibentrocknern, durchgeführt. Geeignete Trockner sind beispielsweise Bepex-Trockner und Nara-Trockner. Überdies können auch Wirbelschichttrockner eingesetzt werden.
Die Trocknung kann im Mischer selbst erfolgen, durch Beheizung des Mantels oder Einblasen von Warmluft. Ebenso geeignet ist ein nachgeschalteter Trockner, wie beispielsweise ein Hordentrockner, ein Drehrohrofen oder eine beheizbare Schnecke. Besonders vorteilhaft wird in einem Wirbelschichttrockner gemischt und getrocknet.

Bevorzugte Trocknungstemperaturen liegen im Bereich 170 bis 250°C, bevorzugt 180 bis 220°C, und besonders bevorzugt 190 bis 210°C. Die bevorzugte Verweilzeit bei dieser Temperatur im Reaktionsmischer oder Trockner beträgt vorzugsweise mindestens 10 Minuten, besonders bevorzugt mindestens 20 Minuten, ganz besonders bevorzugt mindestens 30 Minuten.

Das erfindungsgemäße Verfahren ermöglicht die Herstellung wasserabsorbierender Polymerpartikel mit sehr niedrigem Gehalt an Restmonomeren.

Die gemäß dem erfindungsgemäßen Verfahren erhältlichen wasserabsorbierenden Polymerpartikel weisen eine Zentrifugenretentionskapazität (CRC) von typischerweise mindestens 15 g/g, vorzugsweise mindestens 20 g/g, bevorzugt mindestens 25 g/g, besonders bevorzugt mindestens 30 g/g, ganz besonders bevorzugt mindestens 35 g/g, auf. Die Zentrifugenretentionskapazität (CRC) der wasserabsorbierenden Polymerpartikel beträgt üblicherweise weniger als 100 g/g. Die Zentrifugenretentionskapazität der wasserabsorbierenden Polymerpartikel wird gemäß der von der EDANA (European Disposables and Nonwovens Association) empfohlenen Testmethode Nr. 441.2-02 "Centrifuge retention capacity" bestimmt.

Die gemäß dem erfindungsgemäßen Verfahren erhältlichen wasserabsorbierenden Polymerpartikel weisen einen Gehalt an Restmonomen von typischerweise weniger als 0,1 Gew.-%, vorzugsweise weniger als 0,07 Gew.-%, besonders bevorzugt weniger als 0,05 Gew.-%, ganz besonders bevorzugt von weniger als 0,04 Gew.-%, auf. Der Gehalt an Restmonomeren wird gemäß der von der EDANA (European Disposables and Nonwovens Association) empfohlenen Testmethode Nr. 410.2-02 "Residual monomers" bestimmt.

Die gemäß dem erfindungsgemäßen Verfahren erhältlichen wasserabsorbierenden Polymerpartikel weisen einen Wassergehalt von vorzugsweise mindestens 10 Gew.-%, besonders bevorzugt von mindestens 12 Gew.-%, ganz besonders bevorzugt von mindestens 14 Gew.-%, auf. Der Wassergehalt wird gemäß der von der EDANA (European Disposables and Nonwovens Association) empfohlenen Testmethode Nr. 430.2-02 "Moisture content" bestimmt.

Der mittlere Durchmesser der gemäß dem erfindungsgemäßen Verfahren erhältlichen wasserabsorbierenden Polymerpartikel beträgt vorzugsweise mindestens 200 µm, besonders bevorzugt von 250 bis 600 µm, ganz besonders von 300 bis 500 µm, wobei der Partikeldurchmesser durch Lichtstreuung bestimmt werden kann und den volumengemittelten mittleren Durchmesser bedeutet. 90% der Polymerpartikel weisen einen Durchmesser von vorzugsweise 100 bis 800 µm, besonders bevorzugt von 150 bis 700 µm, ganz besonders bevorzugt von 200 bis 600 µm, auf.

Ein weiterer Gegenstand der vorliegenden Erfindung sind gemäß dem erfindungsgemäßen Verfahren erhältliche wasserabsorbierende Polymerpartikel.
Die wasserabsorbierenden Polymerpartikel werden mittels der nachfolgend beschriebenen Testmethoden geprüft.

Methoden:
Die Messungen sollten, wenn nicht anders angegeben, bei einer Umgebungstemperatur von 23 ± 2 °C und einer relativen Luftfeuchte von 50 ± 10 % durchgeführt werden. Die wasserabsorbierenden Polymere werden vor der Messung gut durchmischt.

### Restmonomer (Residual Monomers)

Der Gehalt an Restmonomer der wasserabsorbierenden Polymerpartikel wird gemäß der von der EDANA (European Disposables and Nonwovens Association) empfohlenen Testmethode Nr. 410.2-02 "Residual monomers" bestimmt.

### Wassergehalt (Moisture Content)

Der Wassergehalt der wasserabsorbierenden Polymerpartikel wird gemäß der von der EDANA (European Disposables and Nonwovens Association) empfohlenen Testmethode Nr. 430.2-02 "Moisture content" bestimmt.

Die EDANA-Testmethoden sind beispielsweise erhältlich bei der European Disposables and Nonwovens Association, Avenue Eugene Plasky 157, B-1030 Brüssel, Belgien.

### Beispiele:

### Beispiel 1 (Vergleichsversuch)

14,3 kg Natriumacrylat (37,5 gew.-%ige Lösung in Wasser), 1,4 kg Acrylsäure und 350 g Wasser wurden mit 22 g 15-fach ethoxiliertem Trimethylolpropantriacrylat gemischt. Als Initiator wurde eine 3 gew.-%ige Lösung von 2,2'-Azobis[2-(2-imidazolin-2-yl)propan]dihydrochlorid in Wasser verwendet. Der Initiator wurde vor einem Vertropfer über einen statischen Mischer in die Monomerlösung dosiert. Die Vertropferplatte hatte 30 Bohrungen à 200 µm. Das Verhältnis von Monomerlösung zu Initiatorlösung betrug 93,6 : 6,4. Die erhaltene Mischung wurde in einen erwärmten, mit Stickstoffatmosphäre gefüllten Vertropfungsturm vertropft (12m Höhe, 2m Breite, Gasgeschwindigkeit 0,1 m/s im Gleichstrom). Die Dosiergeschwindigkeit der Mischung betrug 16 kg/h. Die Heizleistung der Gasvorwärmung wurde so geregelt, dass die Gasausgangstemperatur im Vertropfungsturm konstant 130°C betrug. Die eingespeiste Stickstoffmenge betrug 1.000 m³/h.

Die erhaltenen Polymerpartikel hatten einen Wassergehalt von 19,1 Gew.-%. Die Polymerpartikel enthielte noch 0,5 Gew.-% Restmonomer.

Anschließend wurden die erhaltenen wasserabsorbierenden Polymerpartikel eine Stunde bei 165°C im Umlufttrockenschrank getrocknet. Die getrockneten Polymerpartikel enthielten 0,4 Gew.-% Restmonomer.

### Beispiel 2

Es wurde verfahren wie unter Beispiel 1. Die erhaltenen Polymerpartikel wurde vor der Trocknung in einer Wirbelschicht thermisch nachbehandelt.

Dazu wurden die Polymere 30 Minuten von unten mit einem Luft/WasserdampfGemisch angeströmt. Die Gasgeschwindigkeit betrug 1 m/s. Die Gastemperatur betrug 90°C. Die relative Feuchte des Gasstromes betrug 50%. Der Gasstrom enthielt 0,347 kg Wasserdampf pro kg trockenes Gas.

Die getrockneten Polymerpartikel enthielten 0,3 Gew.-% Restmonomer. Der Wassergehalt der getrockneten Polymerpartikel betrug 5,1 Gew.-%.

### Beispiel 3

Es wurde verfahren wie unter Beispiel 1. Die erhaltenen Polymerpartikel wurde vor der Trocknung in einer Wirbelschicht thermisch nachbehandelt.

Dazu wurden die Polymer 30 Minuten von unten mit einem Luft/Wasserdampf-Gemisch angeströmt. Die Gasgeschwindigkeit betrug 1 m/s. Die Gastemperatur betrug 90°C. Die relative Feuchte des Gasstromes betrug 70%. Der Gasstrom enthielt 0,62 kg Wasserdampf pro kg trockenes Gas.

Die getrockneten Polymerpartikel enthielten 0,08 Gew.-% Restmonomer. Der Wassergehalt der getrockneten Polymerpartikel betrug 11 Gew.-%.

### Beispiel 4

Es wurde verfahren wie unter Beispiel 1. Die erhaltenen Polymerpartikel wurde vor der Trocknung in einer Wirbelschicht thermisch nachbehandelt.

Dazu wurden die Polymer 30 Minuten von unten mit einem Luft/Wasserdampf-Gemisch angeströmt. Die Gasgeschwindigkeit betrug 1 m/s. Die Gastemperatur betrug 90°C. Die relative Feuchte des Gasstromes betrug 90%. Der Gasstrom enthielt 1,1 kg Wasserdampf pro kg trockenes Gas.

Die getrockneten Polymerpartikel enthielten 0,035 Gew.-% Restmonomer. Der Wassergehalt der getrockneten Polymerpartikel betrug 12,3 Gew.-%.

### Beispiel 5

Es wurde verfahren wie unter Beispiel 1. Die erhaltenen Polymerpartikel wurde vor der Trocknung in einer Wirbelschicht thermisch nachbehandelt.

Dazu wurden die Polymer 30 Minuten von unten mit einem Luft/Wasserdampf-Gemisch angeströmt. Die Gasgeschwindigkeit betrug 1,3 m/s. Die Gastemperatur betrug 130°C. Der Gasstrom enthielt 4 kg Wasserdampf pro kg trockenes Gas.

Die getrockneten Polymerpartikel enthielten 0,05 Gew.-% Restmonomer. Der Wassergehalt der getrockneten Polymerpartikel betrug 18,5 Gew.-%.

### Beispiel 6

Es wurde verfahren wie unter Beispiel 1. Die erhaltenen Polymerpartikel wurde vor der Trocknung in einer Wirbelschicht thermisch nachbehandelt.

Dazu wurden die Polymer 30 Minuten von unten mit einem Luft/Wasserdampf-Gemisch angeströmt. Die Gasgeschwindigkeit betrug 0,7 m/s. Die Gastemperatur betrug 110°C. Der Gasstrom enthielt 4 kg Wasserdampf pro kg trockenes Gas.

Die getrockneten Polymerpartikel enthielten 0,03 Gew.-% Restmonomer. Der Wassergehalt der getrockneten Polymerpartikel betrug 15,3 Gew.-%.

## Patentansprüche

1. Verfahren zur Herstellung wasserabsorbierender Polymerpartikel durch Polymerisation von Tropfen einer Monomerlösung, enthaltend
a) mindestens ein ethylenisch ungesättigtes Monomer,
b) wahlweise einen Vernetzer,
c) mindestens einen Initiator und
d) Wasser,
in einer umgebenden Gasphase, **dadurch gekennzeichnet, dass** die erhaltenen Polymerpartikel einen Wassergehalt von mindestens 5 Gew.-% aufweisen, in fluidisiertem Zustand in Gegenwart eines Gasstromes bei einer Temperatur von mindestens 60°C thermisch nachbehandelt werden und die Nachbehandlung mindestens 5 Minuten durchgeführt wird, wobei der Gasstrom bei einer Temperatur von weniger als 100°C eine relative Feuchte von mindestens 20% aufweist oder bei einer Temperatur von 100°C oder mehr mindesten 0,25 kg Wasserdampf pro kg trockenes Gas enthält, wobei die relative Feuchte der Quotient aus Wasserdampfpartialdruck und Wasserdampfdruck (Sättigung) bei einer gegebenen Temperatur multipliziert mit 100% ist, und die Nachbehandlungstemperatur und die relative Feuchte des Gasstromes so gewählt werden, dass sich der Wassergehalt der Polymerpartikel während der Nachbehandlung um weniger als 40 % ändert, wobei die Änderung die relative Änderung bedeutet.

2. Verfahren gemäß Anspruch 1, **dadurch gekennzeichnet, dass** die Nachbehandlungstemperatur und die relative Feuchte des Gasstromes so gewählt werden, dass sich der Wassergehalt der Polymerpartikel während der Nachbehandlung um weniger als 10 % ändert, wobei die Änderung die relative Änderung bedeutet.

3. Verfahren gemäß Anspruch 1 oder 2, **dadurch gekennzeichnet, dass** die Nachbehandlung von 10 bis 30 Minuten durchgeführt wird.

4. Verfahren gemäß einem der Ansprüche 1 bis 3, **dadurch gekennzeichnet, dass** die Nachbehandlung in mindestens einer Wirbelschicht durchgeführt wird.

5. Verfahren gemäß einem der Ansprüche 1 bis 4, **dadurch gekennzeichnet, dass** die Geschwindigkeit des Gasstromes mindestens 0,5 m/s beträgt.

6. Verfahren gemäß einem der Ansprüche 1 bis 5, **dadurch gekennzeichnet, dass** der Wassergehalt der Polymerpartikel während der thermischen Nachbehandlung im Bereich von 5 bis 40 Gew.-% liegt.

7. Verfahren gemäß einem der Ansprüche 1 bis 6, **dadurch gekennzeichnet, dass** Monomer a) zu mindestens 50 mol-% Acrylsäure ist.

8. Verfahren gemäß einem der Ansprüche 1 bis 7 **dadurch gekennzeichnet, dass** die Monomerlösung mindestens 0,1 Gew.-% Vernetzer b), bezogen auf Monomer a) enthält.

9. Verfahren gemäß einem der Ansprüche 1 bis 8, **dadurch gekennzeichnet, dass** die Monomerlösung mindestens eine Azoverbindung und mindestens ein Persulfat als Initiatoren c) enthält.

10. Verfahren gemäß einem der Ansprüche 1 bis 9, **dadurch gekennzeichnet, dass** die Tropfen einen mittleren Durchmesser von mindestens 200 µm aufweisen.

## Claims

1. A process for producing water-absorbing polymer particles by polymerizing droplets of a monomer solution comprising
a) at least one ethylenically unsaturated monomer,
b) optionally a crosslinker,
c) at least one initiator and
d) water,
in a surrounding gas phase, wherein the resulting polymer particles have a water content of at least 5% by weight, are aftertreated thermally in the fluidized state in the presence of a gas stream at a temperature of at least 60°C and the aftertreatment is performed for at least 5 minutes, the gas stream having a relative moisture content of at least 20% at a temperature of less than 100°C or comprising at least 0.25 kg of steam per kg of dry gas at a temperature of 100°C or more, where the relative humidity is the quotient of partial steam pressure and steam pressure (saturation) at a given temperature multiplied by 100%, and the aftertreatment temperature and the relative humidity of the gas stream are selected such that the water content of the polymer particles changes by less than 40% during the aftertreatment, where the change means the relative change.

2. The process according to claim 1, wherein the aftertreatment temperature and the relative humidity of the gas stream are selected such that the water content of the polymer particles changes by less than 10% during the aftertreatment, where the change means the relative change.

3. The process according to claim 1 or 2, wherein the aftertreatment is performed for from 10 to 30 minutes.

4. The process according to any of claims 1 to 3, wherein the aftertreatment is performed in at least one fluidized bed.

5. The process according to any of claims 1 to 4, wherein the velocity of the gas stream is at least 0.5 m/s.

6. The process according to any of claims 1 to 5, wherein the water content of the polymer particles during the thermal aftertreatment is in the range from 5 to 40% by weight.

7. The process according to any of claims 1 to 6, wherein monomer a) is acrylic acid to an extent of at least 50 mol%.

8. The process according to any of claims 1 to 7, wherein the monomer solution comprises at least 0.1% by weight of crosslinker b), based on monomer a) .

9. The process according to any of claims 1 to 8, wherein the monomer solution comprises at least one azo compound and at least one persulfate as initiators c).

10. The process according to any of claims 1 to 9, wherein the droplets have a mean diameter of at least 200 µm.

## Revendications

1. Procédé de fabrication de particules polymères absorbant l'eau par polymérisation de gouttes d'une solution de monomères, contenant :
a) au moins un monomère éthyléniquement insaturé,
b) éventuellement un agent de réticulation,
c) au moins un initiateur et
d) de l'eau,
dans une phase gazeuse entourante, **caractérisé en ce que** les particules polymères obtenues présentent une teneur en eau d'au moins 5 % en poids, sont post-traitées thermiquement à l'état fluidisé en présence d'un courant gazeux à une température d'au moins 60 °C et le post-traitement est réalisé pendant au moins 5 minutes, le courant gazeux présentant à une température inférieure à 100 °C une humidité relative d'au moins 20 %, ou contenant à une température de 100 °C ou plus au moins 0,25 kg de vapeur d'eau par kg de gaz sec, l'humidité relative étant le quotient de la pression partielle de vapeur d'eau et de la pression de vapeur d'eau (saturation) à une température donnée multiplié par 100 %, et la température de post-traitement et l'humidité relative du courant gazeux étant choisies de telle sorte que la teneur en eau des particules polymères se modifie de moins de 40 % pendant le post-traitement, la modification signification la modification relative.

2. Procédé selon la revendication 1, **caractérisé en ce que** la température de post-traitement et l'humidité relative du courant gazeux sont choisies de telle sorte que la teneur en eau des particules polymères se modifie de moins de 10 % pendant le post-traitement, la modification signifiant la modification relative.

3. Procédé selon la revendication 1 ou 2, **caractérisé en ce que** le post-traitement est réalisé pendant 10 à 30 minutes.

4. Procédé selon l'une quelconque des revendications 1 à 3, **caractérisé en ce que** le post-traitement est réalisé dans au moins un lit fluidisé.

5. Procédé selon l'une quelconque des revendications 1 à 4, **caractérisé en ce que** la vitesse du courant gazeux est d'au moins 0,5 m/s.

6. Procédé selon l'une quelconque des revendications 1 à 5, **caractérisé en ce que** la teneur en eau des particules polymères pendant le post-traitement thermique se situe dans la plage allant de 5 à 40 % en poids.

7. Procédé selon l'une quelconque des revendications 1 à 6, **caractérisé en ce que** le monomère a) est l'acide acrylique à hauteur d'au moins 50 % en moles.

8. Procédé selon l'une quelconque des revendications 1 à 7, **caractérisé en ce que** la solution de monomères contient au moins 0,1 % en poids d'agent de réticulation b), par rapport au monomère a).

9. Procédé selon l'une quelconque des revendications 1 à 8, **caractérisé en ce que** la solution de monomères contient au moins un composé azo et au moins un persulfate en tant qu'initiateurs c).

10. Procédé selon l'une quelconque des revendications 1 à 9, **caractérisé en ce que** les gouttes présentent un diamètre moyen d'au moins 200 µm.
